(19) Europäisches Patentamt
European Patent Office
Office européen des brevets

(11) Veröffentlichungsnummer: **0 139 922**
**B1**

(12) # EUROPÄISCHE PATENTSCHRIFT

(45) Veröffentlichungstag der Patentschrift: **08.03.89**

(21) Anmeldenummer: **84109393.3**

(22) Anmeldetag: **08.08.84**

(51) Int. Cl.[4]: **C 08 C 1/14**, C 08 G 18/28, C 08 G 18/10, C 08 G 18/32, C 07 C 127/00

(54) **Wärmesensibilisierungsmittel, ihre Herstellung und Verwendung.**

(30) Priorität: **20.08.83 DE 3330197**

(43) Veröffentlichungstag der Anmeldung: **08.05.85 Patentblatt 85/19**

(45) Bekanntmachung des Hinweises auf die Patenterteilung: **08.03.89 Patentblatt 89/10**

(84) Benannte Vertragsstaaten: **DE FR GB IT**

(56) Entgegenhaltungen:
**DE-B-2 822 908**
**FR-A-2 280 644**

(73) Patentinhaber: **BAYER AG, Konzernverwaltung RP Patentabteilung, D-5090 Leverkusen 1 Bayerwerk (DE)**

(72) Erfinder: **Perrey, Hermann, Dr., Auf der Rheinaue 8, D-4150 Krefeld 11 (DE)**
Erfinder: **Matner, Martin, Dr., Dorfstrasse 14, D-5074 Odenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 139 922 B1

LIBER, STOCKHOLM 1989

## Beschreibung

Wärmesensibilisierte Latexmischungen sind bekannt. Sie werden üblicherweise aus wärmesensibel einstellbaren Polymerlatices hergestellt. Diese Latices können durch Emulsionspolymerisation hergestellt werden. Ihre Wärmesensibilisierung und dazu geeignete Mittel sind z. B. in den deutschen Patentschriften 1 288 828 und 1 494 037 beschrieben.

Ein Verfahren zur Herstellung von wärmesensibilisierbaren Synthesekautschuk-Latices beschreibt die deutsche Patentschrift 1 243 394. Wärmesensibilisierte Latexmischungen können zur Imprägnierung von Faservliesen und zur Herstellung von Hohlkörpern (z. B. Handschuhen) nach dem Tauchverfahren verwendet werden.

Als Wärmesensibilisierungsmittel sind in den aufgeführten Literaturstellen oxalkylierte Polysiloxane beschrieben. Darüber hinaus sind aus zahlreichen Veröffentlichungen weitere Wärmesensibilisierungsmittel, wie Polyvinylalkylether, Polyacetale, kationenaktive Stoffe, Polyetheramine und Polyethylenoxide bekannt. Alle diese Verbindungen besitzen jedoch noch entscheidende Nachteile. Die nachteiligen Eigenschaften der Polyvinylalkylether, der wasserlöslichen Polyacetale, der oxalkylierten Polysiloxane sowie der kationischen Stoffe sind in der deutschen Auslegeschrift 2 228 289, Spalte 1, Zeilen 35 - 53 dargelegt. Die Verwendung der in der Auslegeschrift beschriebenen oxalkylierten Amine ist jedoch durch die Notwendigkeit, einen bestimmten pH-Wert einzustellen, ebenfalls stark eingeengt.

In den deutschen Offenlegungsschriften 2 518 979 und 2 534 304 werden Polyetherurethane als Wärmesensibilisierungsmittel beschrieben, die durch Umsetzung von üblichen Di- und Polyisocyanaten mit vorzugsweise monofunktionellen Polyethern gebildet werden. Mit diesen Produkten können zwar Kautschuklatices wärmesensibel eingestellt werden, bei denen andere Wärmesensibilisierungsmittel versagen, die hierzu benötigten Einsatzmengen liegen jedoch sehr hoch, und so können auch diese Produkte nicht befriedigen.

Überraschenderweise wurde nunmehr gefunden, daß die Wirksamkeit der zuletzt genannten Produkte von polyharnstoffmodifizierten Polyetherurethanen, die zwei oder mehr Harnstoffgruppen enthalten, deutlich übertroffen wird, d. h. die zur Wärmesensibilisierung benötigte Einsatzmenge drastisch veringert werden kann.

Gegenstand der Erfindung sind somit Wärmesensibilisierungsmittel der allgemeinen Formel I

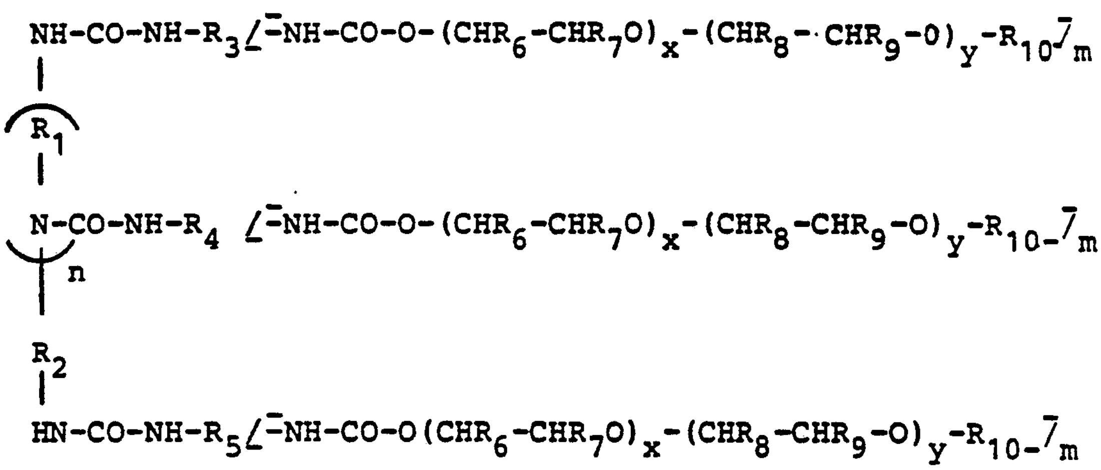

I

in der

$R_1$ und $R_2$ unabhängig voneinander $C_2$ bis $C_4$-Alkylen oder $C_3$ bis $C_{14}$-Cycloalkylen

$R_3$, $R_4$ und $R_5$ unabhängig voneinander gegebenenfalls substituiertes Alkylen, Cycloalkylen oder Arylen,

$R_6$, $R_7$, $R_8$ und $R_9$ unabhängig voneinander Wasserstoff oder Methyl,

$R_{10}$ $C_1$ bis $C_{18}$-Alkyl, $C_8$ bis $C_{18}$-Aryl, $C_7$ bis $C_{18}$-Aralkyl oder $C_2$ bis $C_{18}$-Alkenyl bedeuten,

n für die Zahlen 0 bis 50,

m für die Zahlen 1 bis 4,

x für die Zahlen 5 bis 100 und

y für die Zahlen 0 bis 100 stehen

mit der Maßgabe, daß gilt: $x + y > 10$.

Geeignete Alkylenreste $R_3$, $R_4$ und $R_5$ sind solche mit 1 bis 18 C-Atomen, geeignete Cycloalkylenreste $R_3$, $R_4$ und $R_5$ sind solche mit 5 oder 8 Ringkohlenstoffatomen und geeignete Arylenreste $R_3$, $R_4$ und $R_5$ sind Phenylenund Naphthylenreste, wobei die genannten Reste beispielsweise Urethan-, Uretdion-, Biuret- oder Isocyanursäurereste enthalten können. Die Aryl- und Cycloalkylreste können ferner durch $C_1$-$C_4$-Alkyl oder Chlor substituiert sein.

Bevorzugte Wärmesensibilisierungsmittel entsprechen der Formel II

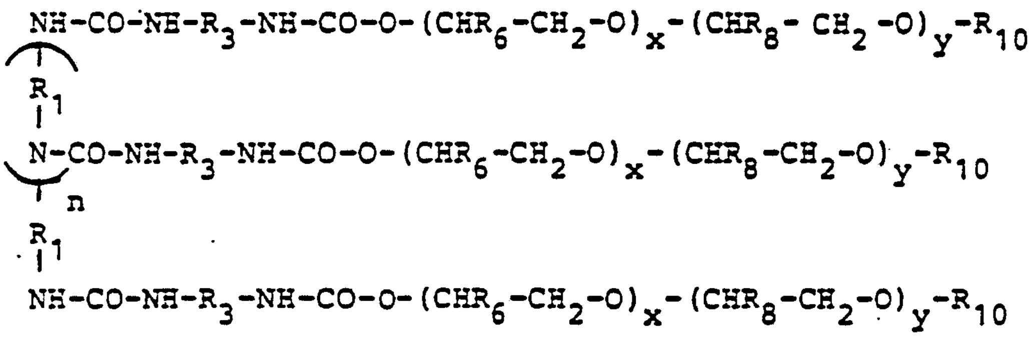

II

in der

$R_1$ $C_2$ bis $C_7$-Alkylen oder $C_5$ bis $C_7$-Cycloalkylen,

$R_3$ $C_6$ bis $C_{20}$-Alkylen oder gegebenenfalls durch $C_1$-$C_4$-Alkyl oder Halogen substituiertes $C_6$-$C_{20}$-Cycloalkylen oder -Arylen,

$R_6$ und $R_8$ Wasserstoff oder Methyl,

$R_{10}$ $C_1$ bis $C_6$-Alkyl bedeuten,

n für die Zahlen 0 bis 5 und

x und y für die Zahlen 5 bis 100 stehen,

wobei die Einheiten ($CHR_6$-$CH_2$-O) und ($CHR_8$-$CH_2$-O) in Blöcken, statistisch verteilt oder teilweise in Blöcken und teilweise statistisch verteilt vorliegen.

Bei ganz besonders bevorzugten Wärmesensibilisierungsmitteln stehen in der Formel II

$R_1$ für $C_2$ bis $C_3$-Alkylen,

$R_3$ für $C_6$ bis $C_{13}$-Alkylen, gegebenenfalls durch Methyl substituiertes $C_6$ bis $C_{13}$-Cycloalkylen oder $C_6$ bis $C_{13}$-Arylen,

$R_6$ für Wasserstoff

$R_8$ für Methyl

n für die Zahlen 1 bis 5,

x für die Zahlen 5 bis 50 und

y für die Zahlen 5 bis 40,

und die Einheiten ($CH_2$-$CH_2$-O) und ($CH(CH_3)$-$CH_2$-O) liegen statistisch oder teilweise in Blöcken und teilweise statistisch verteilt vor.

Die erfindungsgemäßen polyharnstoffmodifizierten Polyetherpolyurethane erhält man durch Umsetzung von (m+1)-wertigen Polyisocyanaten mit Polyethern der Formel (III)

$$HO\text{-}(CHR_6\text{-}CHR_7\text{-}O)_x\text{-}(CHR_8\text{-}CHR_9\text{-}O)_y\text{-}R_{10} \qquad III$$

und Polyaminen der Formel IV

$$NH_2\text{-}(R_1\text{-}NH)_n\text{-}R_2\text{-}NH_2, \qquad IV$$

wobei die Reste $R_1$, $R_2$, $R_6$, $R_7$, $R_8$, $R_9$ und $R_{10}$ und die Zahlen m, n, x und y die oben angegebene Bedeutung besitzen.

Die zur Herstellung der polyharnstoffmodifizierten Polyetherpolyurethane verwendeten Polyisocyanate können aliphatisch, cycloaliphatisch oder aromatisch sein. Als Beispiele für geeignete Polyisocyanate seien Hexamethylendiisocyanat, Cyclohexan-1,4-diisocyanat, 2,4- und 2,6-Toluylendiisocyanat und deren Gemische, 1-Isocyanatomethyl-5-isocyanato-1,3,3-trimethylcyclohexan, (2,2,4-bzw. 2,4,4-Trimethylhexamethylendiisocyanat-1,6), 1,5-Naphthalindiisocyanat, 1,3-Cyclopentylendiisocyanat, m- und p-Phenylendiisocyanat, 2,4,6-Toluylentriisocyanat, 4,4',4''-Triphenylmethantriisocyanat, 1,3- und 1,4-Xylylendiisocyanat, 3,3'-Dimethyl-4,4'-diphenylmethandiisocyanat, 4,4'-Diphenylmethandiisocyanat, 3,3'-Dimethylbiphenylendiisocyanat, 4,4'-Biphenylendiisocyanat, Durendiisocyanat, 1-Phenoxy-2,4'-phenylendiisocyanat, 1-Tert.-butyl-2,4-phenylendiisocyanat, Methylen-bis-4,4'-cyclohexyldiisocyanat, 1-Chlor-2,4-phenylendiisocyanat und 4,4'-Diphenyletherdiisocyanat genannt.

Weiterhin ist es möglich, höhermolekulare und gegebenenfalls auch höherfunktionelle Polyisocyanate, die aus niedermolekularen Grundkörpern durch Polymerisationsreaktion zu Uretdionen oder Isocyanuratderivaten hergestellt werden, einzusetzen. Beispielsweise seien das Uretdion aus 2 Mol 2,4-Toluylendiisocyanat und die isocyanuratringhaltigen Polymerisationsprodukte aus 2,4-und 2,6-Toluylendiisocyanat oder Hexamethylendiisocyanat, ein durchschnittlich 2 Isocyananuratringe im Molekül enthaltenes und aus 5 Mol Toluylendiisocyanat gebildetes System oder ein entsprechendes Derivat aus durchschnittlich 2 Mol Toluylendiisocyanat und 3 Mol Hexamethylendiisocyanat, erwähnt.

Nach einer weiteren Aufbaumethode ist es möglich, aus Di- oder Polyisocyanaten durch partielle Hydrolyse über die Stufe der Carbamidsäure und des Amins höhere biuretverknüpfte Systeme herzustellen, z. B. eine

biuretverknüpfte Verbindung, die formal aus 3 Mol Hexamethylendiisocyanat unter Zusatz von 1 Mol Wasser und Abspaltung von 1 Mol Kohlendioxid entstanden ist.

Ebenfalls geeignete Polyisocyanate erhält man bei der Umsetzung von Di- oder Polyolen mit di- oder polyfunktionellen Isocyanaten, wenn das Molverhältnis von Hydroxyverbindungen zum Isocyanat so gewahlt wird, daß bei den statistisch gebildeten Reaktionsprodukten stets freie NCO-Funktionen vorhanden bleiben und ein Molgewicht von 2000 bis 3000 nicht überschritten wird.

Alle oben beschriebenen Di- und Polyisocyanate konnen in dieser Weise mit Di- und Polyolen, wie Mono- und Polyethylenglykol, Propandiolen, Butandiolen, Neopentylglykol und andere Pentandiolen, Adipol, Hexandiolen, Cyclohexandiolen, 1,4-Dihydroxymethylcyclohexan, Perhydrobisphenol-A, Glycerin, Trimethylolethan, Trimethylolpropan, anderen Hexantriolen und Pentaerythrit, unter den beschriebenen Voraussetzungen umgesetzt werden. Bevorzugt werden die Umsetzungen von Di- und Polyolen mit Toluylendiisocyanat, bei denen pro OH-Funktion 1 Mol des Diisocyanats zur Reaktion kommt.

Bevorzugt werden als Polyisocyanate Diisocyanate, insbesondere Hexamethylendiisocyanat, Isophorondiisocyanat, Toluylendiisocyanat und Diphenylmethandiisocyanat eingesetzt.

Die zur Herstellung der erfindungsgemäßen harnstoffgruppenhaltigen Polyetherurethane verwendeten Polyether erhält man durch Polyalkoxylierung von Alkoholen, Phenolen und Alkylphenolen mit bis zu 18 C-Atomen. Als Alkohole sind hierbei alle gesättigten oder ungesättigten aliphatischen, cycloaliphatischen sowie araliphatischen Hydroxyverbindungen mit 1-18 C-Atomen geeignet, die rein oder in Form von Gemischen zur Polyalkoxylierung eingesetzt werden können.

Als Alkylenoxide zur Polyalkoxylierung kommen z. B. Ethylenoxid, Propylenoxid, 1,2- und 2,3-Epoxybutan oder Epichlorhydrin in Frage.

Bevorzugte Polyether erhält man durch Polyalkoxylierung niederer Alkohole, wie Methanol, Ethanol, Propan-, Butan-, Pentan- oder Hexanole, mit Ethylen- und Propylenoxid. Hierbei können Blockpolymere oder Polymere mit statistischer Verteilung der Oxyalkylgruppen, sog. Mischpolymere, oder auch Mischformen dieser beiden Möglichkeiten hergestellt werden. Bevorzugt sind Mischpolymere und solche Produkte, bei denen die Alkohole zunächst mit einer Mischung aus Propylenoxid und 80 - 90 % der gesamten Menge des Ethylenoxids zu Mischpolymeren umgesetzt werden und hierauf die restlichen 10 - 20 % des Ethylenoxids eingebracht werden, so daß die Endgruppen dieser Polyether nahezu vollständig primäre OH-Gruppen sind. Bevorzugte Polyether enthalten 40 - 60 Gew.-% Ethylenoxid, besonders bevorzugte sind aus gleichen Gewichtsmengen Ethylen- und Propylenoxid aufgebaut. Bevorzugt werden Polyether mit Molekulargewichten von 600 - 5000, besonders bevorzugt mit 700 bis 3000 eingesetzt.

Als zur Herstellung der erfindungsgemäßen polyharnstoffmodifizierten Polyetherpolyurethane geeignete aliphatische oder cycloaliphatische Amine seien Ethylendiamin, Diethylentriamin, Triethylentetramin, Tetraethylenpentamin, Pentaethylenhexamin, Polyethylenimin, 1,2-und 1,3-Propylendiamin, Dipropylentriamin, Tripropylentetramin, Butylendiamin, Hexamethylendiamin, 2,5-Diamino-2,5-dimethylhexan, 2,2,4- und 2,4,4-Trimethyl-1,6-hexandiamin, Cyclohexandiamin, Isophorondiamin, hydrierte Toluylendiamine und hydrierte Diaminodiphenylmethane genannt.

Bevorzugte Polyamine sind tri- und höherfunktionell. Diethylentriamin, Triethylentetramin, Tetraethylenpentamin und Pentaethylenhexamin sind besonders bevorzugt.

Die Umsetzung der Polyisoycanate mit den Polyethern und den Polyaminen kann zwar so durchgeführt werden, daß zu dem vorgelegten Polyisocyanat eine Mischung aus Polyether und Polyamin getropft wird oder umgekehrt zu einer Mischung aus Polyether und Polyamin das Polyisocyanat getropft wird, doch führen diese Verfahrensweisen häufig zu unlöslichen Ausscheidungen, daher wird vorzugsweise 2-stufig gearbeitet. Zunächst wird der Polyether mit dem Polyisocyanat umgesetzt und hierauf die restlichen Isocyanatgruppen mit dem Polyamin zur Reaktion gebracht.

Bei der Umsetzung des Polyethers mit dem Polyisocyanat wird vorteilhaft das Polyisocyanat vorgelegt und der Polyether zugetropft. Zur Herstellung reproduzierbarer Produkte ist es wichtig, daß die Polyether stets wasserfrei eingesetzt werden. Die Reaktion kann in inerten Lösungsmitteln, wie Benzol, Toluol, Xylol, Chlorbenzol, o-Dichlorbenzol, Aceton und Essigester oder auch lösungsmittelfrei durchgeführt werden; vorzugsweise wird im Temperaturbereich zwischen 0 und 140°C gearbeitet. Alle bei der Urethanbildung wirksamen Katalysatoren (s. Houben-Weyl, Methoden der organischen Chemie, Bd. 14.2, Seite 61, 4. Auflage, 1963), wie Pyridin, Methylpyridin, N,N'-Dimethylpiperazin, N,N-Dimethylbenzylamin oder N,N'-Endoethylenpiperazin, können verwendet werden.

Bei den Formeln I und II handelt es sich um idealisierte Strukturen. Je nach der Reaktivität der Reaktionsteilnehmer kommt es zur Ausbildung von Polymergemischen. Werden allerdings Diisocyanate mit Isocyanatgruppen unterschiedlicher Reaktivität eingesetzt, wie 2,4-Toluylendiisocyanat oder Isophorondiisocyanat, so ist es bei der Umsetzung mit den Polyethern möglich, in hohen Ausbeuten zunächst nur die reaktivere Isocyanatgruppe des Molekuls umzusetzen. Die als Nebenreaktion mögliche Bildung des Reaktionsproduktes aus 2 Mol Polyether und 1 Mol Diisocyanat neben einem Mol freiem Diisocyanat kann durch schonende Reaktionsbedingungen in erheblichem Maße unterdrückt werden, so daß nach der Umsetzung mit den Polyaminen weitgehend die Verbindungen gemäß Formeln I und II erhalten werden.

Reaktivität eingesetzt, wie Hexamethylendissocyanat oder 4,4'-Diphenylmethandiisocyanat, so kommt es bei der Reaktion mit dem Polyether zu einem Reaktionsgemisch aus 1 : 1-, 1 : 2-Additionsprodukt und unumgesetztem Diisocyanat, das nach Reaktion mit dem Polyamin somit zu einem Produktgemisch aus dem 1 : 2-Additionsprodukt aus 1 Mol Diisocyanat und 2 Mol Polyether, aus Verbindungen gemäß Formel I bzw. II und

insbesondere auch aus höhermolekularen Bestandteilen führt. Diese höhermolekularen Bestandteile werden dadurch aufgebaut, daß Molekülteile, bei denen nicht alle NH-Gruppen des Polyamins über Diisocyanate mit Polyethern verbunden sind, über die noch nicht umgesetzten NH-Gruppen mit den freien Diisocyanaten zu höhermolekularen Polyharnstoffen reagieren. Produkte bei denen die stöchiometrischen Verhältnisse bis zu 30 % von den durch die Formeln angegebenen Verhältnissen abweichen, sind aber immer noch gut geeignet. Hierbei sind die stöchiometrischen Verhältnisse bevorzugt, bei denen die mölmäßige Summe der OH- und NH-Gruppen gleich der der Isocyanatgruppen ist.

In der Regel liegen die erfindungsgemäßen Verbindungen als wasserlösliche oder wasseremulgierbare Öle vor. Da ihre Wirksamkeit als Wärmesensibilisierungsmittel mit guter Verteilung steigt, ist es manchmal zur Verbesserung der Verteilbarkeit angebracht, gängige Emulgatoren, z. B. Alkylarylsulfonate, Alkylsulfate, Fettsäuresalze, Alkylphenol-, Fettalkoholethoxylate oder dergleichen zuzufügen.

Zur Herstellung der wärmesensibilisierbaren stabilen Latices selbst können übliche olefinisch ungesättigte Monomere in wäßriger Emulsion polymerisiert werden. Als Monomere kommen alle radikalisch polymerisierbaren olefinisch ungesättigten Verbindungen in Frage, z. B. Ethylen, Butadien, Isopren, Acrylnitril, Styrol, Divinylbenzol, α-Methylstyrol, Methacrylnitril, Acrylsäure, Methacrylsäure, 2-Chlorbutadien-1,3, Ester der Acrylsäure und Methacrylsäure mit $C_1$ bis $C_8$-Alkoholen oder Polyolen, Acrylamid, Methacrylamid, N-Methylol(meth)acrylamid, (Meth)acrylamido-N-methylolmethylether, Itakonsäure, Maleinsäure, Fumarsäure, Diester und Halbester ungesättigter Dicarbonsäuren, Vinylchlorid, Vinylacetat, Vinylidenchlorid, die allein oder in Kombination miteinander eingesetzt werden können.

Die Polyermisation wird in Gegenwart von Emulgatoren durchgeführt, wobei die üblichen nichtionischen oder anionischen Emulgiermittel allein oder in Kombination miteinander verwendet werden können. Die Gesamtmenge an Emulgator beträgt ca. 0,1 - 10 Gew.-%, bezogen auf die Monomeren.

Die Emulsionspolymerisation kann mit Radikalbildnern, vorzugsweise mit organischen Peroxidverbindungen ausgelöst werden, die in Mengen von 0,01 bis 2 Gew.-%, bezogen aut Monomere, eingesetzt werden. Je nach Monomerkombination konnen zur Erniedrigung des Molekulargewichtes des Polymerisats geringe Mengen an Reglern mitverwendet werden, z. B. Mercaptane oder Halogenkohlenwasserstoffe. Die Emulsionspolymerisation ist auf zwei Wegen möglich: Man kann die Gesamtmenge der Monomeren und den größten Teil der die Emulgatoren enthaltenden wäßrigen Phase vorlegen, die Polymerisation durch Zugabe des Initiators starten und im Verlauf der Polymerisation den Rest der wäßrigen Phase kontinuierlich oder absatzweise zugeben. Man kann auch die Technik des "Monomerenzulaufs" benutzen; dabei wird nur ein Teil der Monomeren und der das Emulgiermittel enthaltenden wäßrigen Phase vorgelegt und nach Starten der Polymerisation der Rest der Monomeren und der wäßrigen Phase gleichmäßig oder absatzweise nach Maßgabe des Umsatzes zugefügt. Der zudosierte Monomerenanteil kann in der wäßrigen Phase voremulgiert sein. Beide Verfahren sind bekannt.

Die wärmesensibel einstellbaren Latices können vor oder bei der Verarbeitung mit Zusatzstoffen versetzt werden. So unterstützen Säureabspalter, die zusätzlich zum Sensibilisierungsmittel beigefügt werden, die Koagulierfähigkeit, indem sie die Koagulationstemperatur herabsetzen. Andere Zusätze sind z. B. Farbstoffe, Pigmente, Füllstoffe, Verdicker, Elektrolyte, Alterungsschutzmittel, wasserlösliche Harze oder Vulkanisationschemikalien.

Im Anschluß an die Herstellung werden die wärmesensibilisierbaren Latices durch Zusatz der erfindungsgemäßen Verbindungen in Mengen von 1 - 20 Gew.-%, vorzugsweise 2 - 15 Gew.-% bezogen auf das Polymerisat, wärmesensibel eingestellt. Hierbei kann die Zugabe der Produkte in 100 %-iger oder häufig vorteilhafter als wäßrige Lösung erfolgen. Es zeigt sich, daß die wärmesensiblen Latexmischungen, die die erfindungsgemäßen Produkte enthalten, auch bei längerer Lagerung stabil sind und keine Koagulatbildung zeigen.

Die erfindungsgemäßen, wärmesensibel eingestellten Latexmischungen können z. B. zur Bindung von Faservliesen eingesetzt werden, die aus synthetischen oder natürlichen Fasern aufgebaut sind.

In vielen Fällen erweist es sich als günstig, die benötigte Menge des Wärmesensibilisierungsmittels durch Zusatz von Elektrolyten zu reduzieren. Geeignete Elektrolyte sind z. B. NaCl, KCl, $NH_4Cl$, Na-Acetat, K-Acetat und $NH_4$-Acetat, von denen 0,1 bis 10 Gew.-% bezogen auf das Polymerisat, eingesetzt werden.

Der besondere Vorteil der erfindungsgemäßen Wärmesensibilisierungsmittel liegt darin, daß die elektrolythaltigen Latexmischungen außerordentlich stabil bei Lagerung und mechanischer Belastung sind und nicht zu vorzeitiger Koagulation neigen.

**Beispiel 1**

In eine Lösung aus 174 g (1 Mol) 2,4-Toluylendiisocyanat und 0,17 g Dibutyldizinnlaurat fügte man bei 50°C innerhalb einer Stunde 1273 g (1 Mol) eines Polyethers der OH-Zahl 44, der durch Alkoxylierung von Butanol mit gleichen Gewichtsmengen Ethylen- und Propylenoxid hergestellt wurde, wobei zunächst 80 % der Ethylenoxid- mit der gesamten Propylenoxidmenge im Gemisch und hierauf die restlichen 20 % des Ethylenoxids umgesetzt wurden, und ließ ca. 2 h nachreagieren.

Nachdem somit 1 Mol Isocyanat zum Urethan reagiert hatte (Kontrolle über Isocyanatzahlbestimmung)

tropfte man 34,4 g (0,33 Mol) Diethylentriamin bei 50°C hinzu und ließ 2 h nachreagieren. Man isolierte eine schwachgelbe, ölige, in Wasser lösliche Flüssigkeit.

**Beispiel 2** (Vergleichsbeispiel)

In eine Mischung aus 174 g (1 Mol) Toluylendiisocyanat fügte man bei 50°C innerhalb einer Stunde 1273 g (1 Mol) eines in Beispiel 1 beschriebenen Polyethers der OH-Zahl 44 und ließ darauf ca. 2 h nachreagieren. Nachdem nunmehr 1 Mol Isocyanat zum Urethan reagiert hatte, fügte man 1,5 g feingemörsertes Kaliumacetat hinzu und ließ ca. 10 h bei 50°C reagieren. Unter diesen Reaktionsbedingungen reagierte das zweite Mol Isocyant unter Isocyanuratbildung zu einem isocyanatgruppenfreien Produkt. Anstelle der erfindungsgemäßen Polyharnstoffmodifizierung enthielt dieses Produkt somit eine Isocyanuratverknüpfung.

**Beispiel 3** (Vergleichsbeispiel)

Man verfuhr wie in Beispiel 1 beschrieben, fügte jedoch anstelle von 0,33 Mol Diethylentriamin 44,7 g (0,33 Mol) Trimethylolpropan hinzu. Das erhaltene Reaktionsprodukt enthielt nicht die erfindungsgemäße Polyharnstoffmodifizierung.

**Beispiel 4**

Analog Beispiel 1 wurden anstelle von Toluylendiisocyanat 221 g (1 Mol) 1-Isocyanatomethyl-5-isocyanato-1,3,3-trimethylcyclohexan eingesetzt.

**Beispiel 5**

Analog Beispiel 1 wurden anstelle von Toluylendiisocyanat 250 g (1 Mol) 4,4'-Diisocyanatodiphenylmethan eingesetzt.

**Beispiel 6**

Analog Beispiel 1 wurden anstelle von 0,33 Mol Diethylentriamin 38,7 g (0,167 Mol) Pentaethylenhexamin eingesetzt. Man erhält ein bei Zimmertemperatur hochviskoses Öl.

**Beispiel 7**

Analog Beispiel 1 wurden anstelle des Polyethers der OH-Zahl 44 1870 g (1 Mol) Polyether der OH-Zahl 30 eingesetzt, der ebenfalls durch Alkoxylierung von Butanol mit gleichen Gewichtsmengen Ethylen- und Propylenoxid hergestellt wurde, wobei zunächst 80 % der Ethylenoxid- mit der gesamten Propylenoxidmenge im Gemisch und hierauf die restlichen 20 % des Ethylenoxids umgesetzt wurden.

Das Reaktionsprodukt löste sich klar in Wasser.

**Beispiel 8**

Unter den in Beispiel 1 beschriebenen Reaktionsbedingungen wurden zunächst 168 g (1 Mol) Hexamethylendiisocyanat mit 801 g (1 Mol) eines Polyethers der OH-Zahl 70, der durch Alkoxylierung von Butanol mit gleichen Gewichtsmengen Ethylen- und Propylenoxid hergestellt wurde, und hierauf mit 34,4 g (0,33 Mol) Diethylentriamin umgesetzt.

**Beispiel 9**

Unter den in Beispiel 1 beschriebenen Reaktionsbedingungen wurden zunächst 168 g (1 Mol) Hexamethylendiisocyanat mit 1273 g (1 Mol) des in Beispiel 1 beschriebenen Polyethers der OH-Zahl 44 und hierauf mit 34,4 g (0,33 Mol) Diethylentriamin umgesetzt. Das Reaktionsprodukt löste sich klar in Wasser.

**Beispiel 10** (Vergleichsbeispiel)

Man verfuhr entsprechend Beispiel 9, verwendete jedoch anstelle des Diethylentriamins 44,7 g (0,33 Mol) Trimethylolpropan. Das Reaktionsprodukt enthielt somit nicht die erfindungsgemäße Polyharnstoffmodifizierung, sondern eine Urethanverknüpfung.

**Beispiel 11** (Vergleichsbeispiel)

159,3 g (0,33 Mol) eines biuretverknüpften Triisocyanats, das formal aus 3 Mol Hexamethylendiisocyanat und 1 Mol Wasser unter Abspaltung von 1 Mol $CO_2$ entstanden war, wurde mit 1273 g (1 Mol) des in Beispiel 1 beschriebenen Polyethers der OH-Zahl 44 unter den in Beispiel 1 beschriebenen Reaktionsbedingungen umgesetzt. Das erhaltene Reaktionsprodukt enthielt anstelle der erfindungsgemaßen Polyharnstoff- eine Biuretverknüpfung.

**Beispiel 12 - 18**

224 Gew.-Teile eines 40 %-igen Latex des Copolymerisates von 64 Gew.-% Butadien, 31 Gew.-% Acrylnitril, 3,5 Gew.-% N-Methylolacrylamid und 1,5 Gew.-% Acrylamid wurden mit 64 Gew.-Teilen destilliertem Wasser, 10 Gew.-Teilen 10 %-igen Natriumhexametaphosphat-Lösung und 60 Gew.-Teilen eines Wärme sensibilisierungsmittels vermischt und der Koagulationspunkt der Mischungen nach 30 Minuten und nach 7 Tagen Standzeit bestimmt.

Methode zur Ermittlung des Koagulationspunktes:

10 g der wärmesensibel eingestellten Mischung wurden in ein Becherglas eingewogen und in ein Wasserbad mit einer konstanten Temperatur von 80°C gebracht. Unter gleichmäßigem Rühren der Mischung mit einem Thermometer wurden das Koagulierverhalten und der Temperaturanstieg verfolgt. Als Koagulationspunkt der Mischung wurde die Temperatur angegeben, bei der eine vollständige und endgültige Trennung von Polymerisat und wäßriger Phase eintrat.

| Beispiel | Wärmesensibiliserungs-mittel gemäß Beispiel | Koagulationspunkt (°C) nach | |
|---|---|---|---|
| | | 30 Minuten | 7 Tage |
| 12 | 1 | 43 | 42 |
| 13 | 4 | 49 | 48 |
| 14 | 5 | 44 | 42 |
| 15 | 6 | 41 | 37 |
| 16 | 7 | 47 | 47 |
| 17 | 8 | 35 | 34 |
| 18 | 9 | 41 | 40 |

**Beispiel 19 und 20** (Vergleichsbeispiele)

In diesen Beispielen wurden die Einsatzmengen der Produkte aus Beispielen 2 und 3 ermittelt, die zur Einstellung des gleichen Koagulationspunktes erforderlich sind, wie er sich bei der Latexmischung gemäß Beispiel 12 aus 6 g des erfindungsgemäßen Wärmesensibilisierungsmittels aus Beispiel 1 ergab.

| Beispiel | Einsatzmenge (g) | Verbindung gemäß Beispiel | Koagulationspunkt (°C) |
|---|---|---|---|
| 19 | 11 | 2 | 43 |
| 20 | 13 | 3 | 43 |
| 12 | 6 | 1 | 43 |

Diese Beispiele belegen deutlich die besondere Wirksamkeit der erfindungsgemäßen Polyharnstoffmodifizierung gegenuber der Isocyanat- bzw. Urethanverknüpfung.

**Beispiele 21 und 22** (Vergleichsbeispiele)

In diesen Beispielen wurden die für einen Koagulationspunkt von 41°C erforderlichen Mengen an Wärmesensibilisierungsmittel gemäß Beispielen 10 und 11 ermittelt, wobei auch hier die Latexmischung gemäß Beispielen 12 - 18 verwendet wurde.

| Beispiel | Einsatzmenge (g) | Verbindung gemäß Beispiel | Koagulationspunkt (°C) |
|---|---|---|---|
| 21 | 14 | 10 | 41 |
| 22 | 12 | 11 | 41 |
| 18 | 6 | 9 | 41 |

Auch diese Beispiele belegen deutlich die besondere Wirksamkeit der erfindungsgemäßen polyharnstoffmodifizierten Polyurethane gegenüber sonst vom Aufbau vergleichbaren urethan- bzw. biuretverknüpften Typen.

**Beispiel 23**

Zur Herstellung einer wärmesensibel verarbeitbaren Latexmischung wurden folgende Bestandteile zusammengerührt:

225,0 Gew.-Teile eines 45 gew.-%-igen Latex des Copolymerisats von
66,0 Gew.-% Butadien,
30,0 Gew.-% Acrylnitril und
4,0 Gew.-% Methacrylsäure
40,0 Gew.-Teile einer Vulkanisationspaste aus
0,2 Gew.-Teilen Kolloidschwefel,
0,2 Gew.-Teilen Zink-N,N'-diethyldithiocarbamat,
1,5 Gew.-Teilen Zink-mercaptobenzthiazol,
5,0 Gew.-Teilen Zinkoxid,
5,0 Gew.-Teilen Titandioxid,
28,1 Gew.-Teilen einer 5 %-igen wäßrigen Lösung eines Kondensationsproduktes aus Naphthalinsulfonsäure mit Formaldehyd,
2,0 Gew.-Teile einer 25 gew.-%-igen wäßrigen Ammoniaklösung,
54,0 Gew.-Teile einer 40 gew.-%-igen Dispersion von Calciumcarbonat in Wasser,
84,0 Gew.-Teile Wasser
12,0 Gew.-Teile des unter Beispiel 1 beschriebenen Polyetherurethans.

Der nach der beschriebenen Methode gemessene Koagulationspunkt der Latexmischung betrug 40°C und war über 7 Tage konstant.

**Beispiel 24**

Es wurde eine Latexmischung analog Beispiel 23 hergestellt. Als Wärmesensibilisierungsmittel wurden hier jedoch 7,0 Gew.-Teile des Polyetherpolyurethans des Beispiels 8 eingesetzt.

Die Latexmischung besaß einen Koagulationspunkt von 43°C, der sich über mehrere Tage nicht veränderte.

**Beispiel 25**

Zu den 232,0 Gew.-Teilen eines 43,5 gew.-%-igen Latex eines Copolymerisats von 46,0 Gew.-% Styrol, 50,0 Gew.-% Butadien, 2,0 Gew.-% Acrylsäure und 2,0 Gew.-% N-Methylolacrylamid gibt man 62,0 Gew.-Teile Wasser und 10,0 Gew.-Teile des im Beispiel 1 beschriebenen Polyetherpolyurethans.

Der Koagulationspunkt der auf diese Weise hergestellten, wärmesensibilisierbaren Latexmischung lag bei 38° C und war über mehrere Tage konstant.

**Beispiel 26**

173,0 Gew.-Teile eines 58 gew.-%igen Polychloropren-Latex,
35,5 Gew.-Teile einer Vulkanisationspaste aus
7,5 Gew.-Teilen Zinkoxid,
2,0 Gew.-Teilen Diphenylthioharnstoff,
1,0 Gew.-Teilen Diphenylguanidin,
1,0 Gew.-Teilen Schwefel und
24,0 Gew.-Teilen einer 5 gew.-%-igen wäßrigen Lösung eines Kondensationsproduktes aus Naphthalinsulfonsäure mit Formaldehyd,
35,0 Gew.-Teile Wasser,
10,0 Gew.-Teile einer 10 gew.-%-igen wäßrigen Ammoniumchloridlösung und
4,0 Gew.-Teile des in Beispiel 1 beschriebenen Polyetherpolyurethans wurden zusammengerührt.
Diese wärmesensibel eingestellte Latexmischung besaß eine Koagulationstemperatur von 42° C.

**Patentansprüche**

1. Wärmesensibilisierungsmittel der allgemeinen Formel

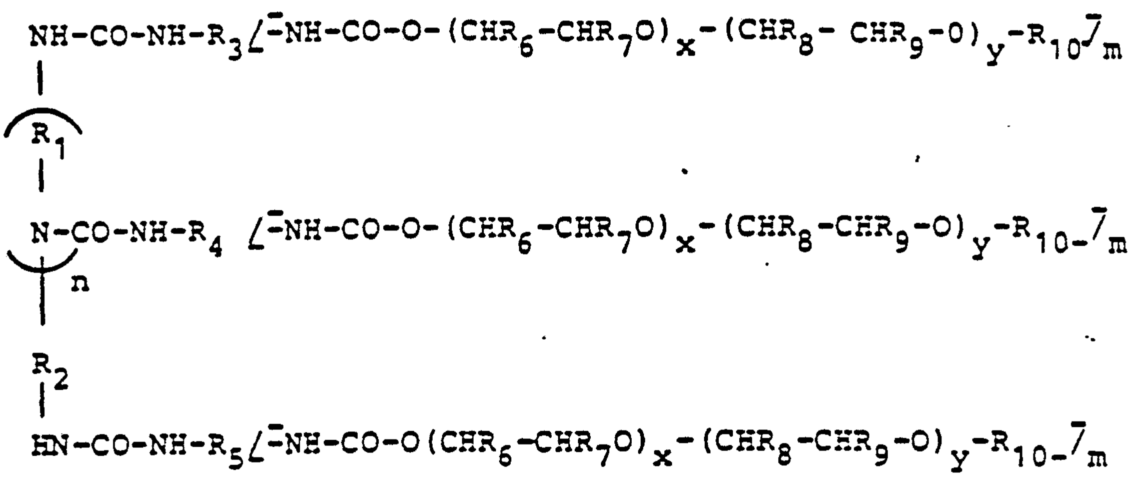

in der
$R_1$ und $R_2$ unabhängig voneinander $C_2$ bis $C_{14}$-Alkylen oder $C_3$ bis $C_{14}$-Cycloalkylen,
$R_3$, $R_4$ und $R_5$ unabhängig voneinander gegebenenfalls substituiertes Alkylen, Cycloalkylen oder Arylen,
$R_6$, $R_7$, $R_8$ und $R_9$ unabhängig voneinander Wasserstoff oder Methyl,
$R_{10}$ $C_1$ bis $C_{18}$-Alkyl, $C_6$ bis $C_{18}$-Aryl, $C_7$ bis $C_{18}$-Aralkyl oder $C_2$ bis $C_{18}$-Alkenyl bedeuten,
n für die Zahlen 0 bis 50,
m für die Zahlen 1 bis 4,
x für die Zahlen 5 bis 100 und
y für die Zahlen 0 bis 100 stehen
mit der Maßgabe, daß gilt: $x + y > 10$.

2. Wärmesensibilisierungsmittel der allgemeinen Formel

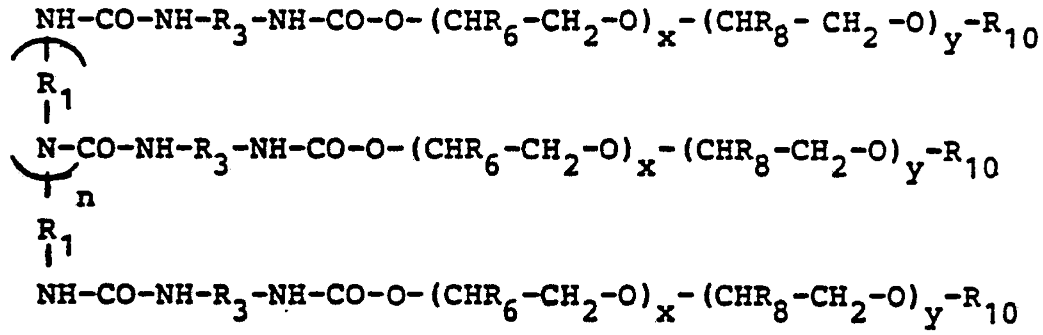

in der

$R_1$ $C_2$ bis $C_7$-Alkylen oder $C_5$ bis $C_7$-Cycloalkylen,

$R_3$ $C_6$ bis $C_{20}$-Alkylen oder gegebenenfalls durch $C_1$-$C_4$-Alkyl oder Halogen substituiertes $C_6$-$C_{20}$-Cycloalkylen oder -Arylen,

$R_6$ und $R_8$ Wasserstoff oder Methyl,

$R_{10}$ $C_1$ bis $C_6$-Alkyl bedeuten,

n für die Zahlen 0 bis 5 und

x und y für die Zahlen 5 bis 100 stehen,

wobei die Einheiten ($CHR_6$-$CH_2$-O) und ($CHR_8$-$CH_2$-O) in Blöcken, statistisch verteilt oder teilweise in Blöcken und teilweise statistisch verteilt vorliegen.

3. Wärmesensibilisierungsmittel gemäß Anspruch 2, wobei

$R_1$ für $C_2$ bis $C_3$-Alkylen,

$R_3$ für $C_6$ bis $C_{13}$-Alkylen, gegebenenfalls durch Methyl substituiertes $C_6$ bis $C_{13}$-Cycloalkylen oder $C_6$ bis $C_{13}$-Arylen

$R_6$ für Wasserstoff

$R_8$ für Methyl

n fur die Zahlen 1 bis 5,

x für die Zahlen 5 bis 50 und

y für die Zahlen 5 bis 40 stehen,

und die Einheiten ($CH_2$-$CH_2$-O) und ($CH(CH_3)$-$CH_2$-O) statistisch oder teilweise in Blöcken und teilweise statistisch verteilt vorliegen.

4. Verfahren zur Herstellung von Verbindungen nach Anspruch 1, dadurch gekennzeichnet, daß man (m+1)-wertige Polyisocyanate mit Polyethern der Formel

$$HO\text{-}(CHR_6\text{-}CHR_7\text{-}O)_x\text{-}(CHR_8\text{-}CHR_9\text{-}O)_y\text{-}R_{10}$$

und Polyaminen der Formel

$$NH_2\text{-}(R_1\text{-}NH)_n\text{-}R_2\text{-}NH_2,$$

wobei die Reste $R_1$, $R_2$, $R_6$, $R_7$, $R_8$, $R_9$ und $R_{10}$ und die Zahlen m, n, x und y die oben angegebene Bedeutung besitzen, umsetzt.

5. Verfahren zur Herstellung wärmesensibel eingestellter Latices, dadurch gekennzeichnet, daß man den Latices Verbindungen nach Anspruch 1 zusetzt.

6. Verfahren nach Anspruch 5, dadurch gekennzeichnet, daß die Wärmesensibilisierungsmittel in Mengen von 1 bis 20 Gew.-%, bezogen auf Polymerisat, dem Latex zugesetzt werden.

**Claims**

1. Heat sensitizers corresponding to the following general formula

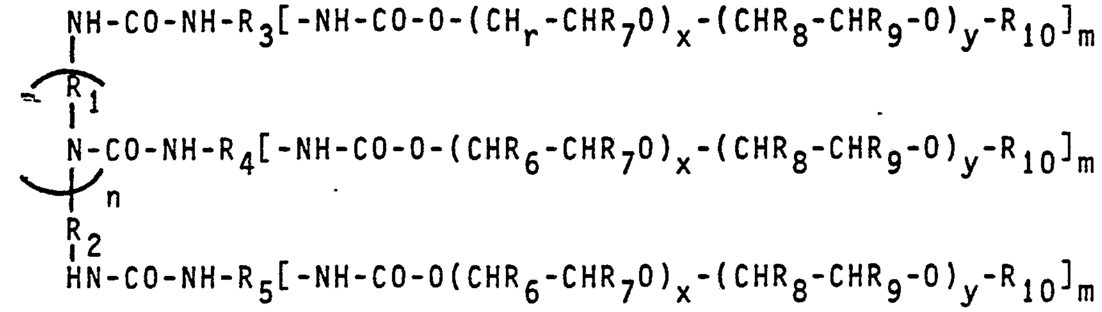

in which
$R_1$ and $R_2$ independently of one another represent $C_2$-$C_{14}$ alkylene or $C_3$-$C_{14}$ cycloalkylene,
$R_3$, $R_4$ and $R_5$ independently of one another represent optionally substituted alkylene, cycloalkylene or arylene,
$R_6$, $R_7$, $R_8$ and $R_9$ independently of one another represent hydrogen or methyl,
$R_{10}$ represents $C_1$-$C_{18}$ alkyl, $C_6$-$C_{18}$ aryl, $C_7$-$C_{18}$ aralkyl or $C_2$-$C_{18}$ alkenyl,
n stands for the numbers 0 to 50,
m stands for the numbers 1 to 4,
x stands for the numbers 5 to 100 and
y stands for the numbers 0 to 100,
with the proviso that $x + y > 10$

2. Heat sensitizers corresponding to the following general formula

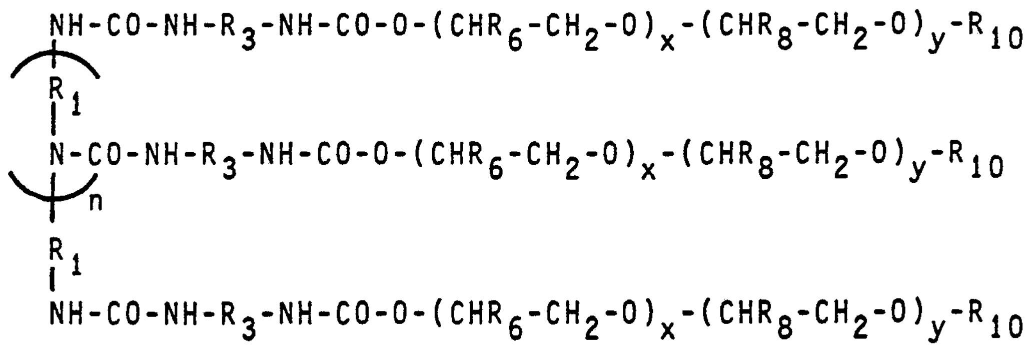

in which
$R_1$ represents $C_2$-$C_7$ alkylene or $C_5$-$C_7$ cycloalkylene,
$R_3$ represents $C_6$-$C_{20}$ alkylene or $C_6$-$C_{20}$ cycloalkylene or arylene optionally substituted by $C_1$-$C_4$ alkyl or halogen,
$R_6$ and $R_8$ represent hydrogen or methyl,
$R_{10}$ represents $C_1$-$C_6$ alkyl,
n stands for the numbers 0 to 5 and
x and y stand for the numbers 5 to 100,
the units ($CHR_6$-$CH_2$-O) and ($CHR_8$-$CH_2$-O) being present in blocks, in statistical distribution or partly in blocks and partly in statistical distribution.

3. Heat sensitizers as claimed in claim 2, in which
$R_1$ represents $C_2$-$C_3$ alkylene,
$R_3$ represents $C_6$-$C_{13}$ alkylene, optionally methyl-substituted $C_6$-$C_{13}$ cycloalkylene or $C_6$-$C_{13}$ arylene,
$R_6$ represents hydrogen,
$R_8$ represents methyl,
n stands for the numbers 1 to 5,
x stands for the numbers 5 to 50 and
y stands for the numbers 5 to 40,
and the units ($CH_2$-$CH_2$-O) and ($CH(CH_3)$-$CH_2$-O) are present in statistical distribution or partly in blocks and partly in statistical distribution.

4. A process for the production of the compounds claimed in claim 1, characterized in that (m+1)-functional polyisocyanates are reacted with polyethers corresponding to the formula

$$HO\text{-}(CHR_6\text{-}CHR_7\text{-}O)_x\text{-}(CHR_8\text{-}CHR_9\text{-}O)_y\text{-}R_{10}$$

and polyamines corresponding to the formula

$$NH_2\text{-}(R_1\text{-}NH)_n\text{-}R_2\text{-}NH_2,$$

the substituents $R_1$, $R_2$, $R_6$, $R_7$, $R_8$, $R_9$ and $R_{10}$ and the indices m, n, x and y being as defined above.

5. A process for the production of heat-sensitized latices, characterized in that the compounds claimed in claim 1 are added to the latices.

6. A process as claimed in claim 5, characterized in that the heat sensitizers are added to the latex in quantities of from 1 to 20 % by weight, based on polymer.

**Revendications**

1. Agent de thermosensibilisation, de formule générale:

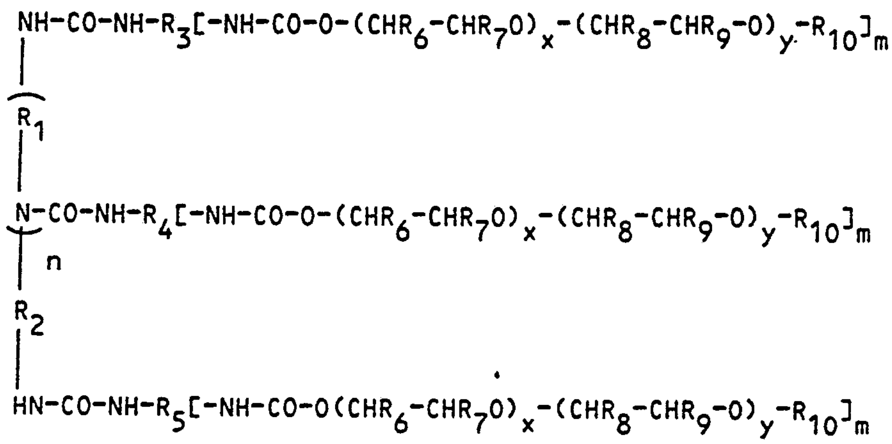

dans laquelle:

$R_1$ et $R_2$ représentent, indépendamment l'un de l'autre, un groupe allkylène en $C_2$ à $C_{14}$ ou un groupe cycloalkylène en $C_3$ à $C_{14}$,

$R_3$, $R_4$ et $R_5$ représentent, indépendamment l'un de l'autre, un groupe alkylène, cycloalkylène ou arylène, évenluellement substitués,

$R_6$, $R_7$, $R_8$ et $R_9$ représentent, indépendamment l'un de l'autre, un atome d'hydrogène ou un groupe méthyle,

$R_{10}$ représente un groupe alkyle en $C_1$ à $C_{18}$, aryle en $C_6$ à $C_{18}$, aralkyle en $C_7$ à $C_{18}$ ou alcényle en $C_2$ à $C_{18}$,

n représente les nombres 0 à 50,

m représente les nombres 1 à 4,

x représente les nombres 5 à 100, et

y représente les nombres 0 à 100,

à la condition que la relation $x + y > 10$ soit valable.

2. Agent de thermosensibilisation, répondant à la formule générale:

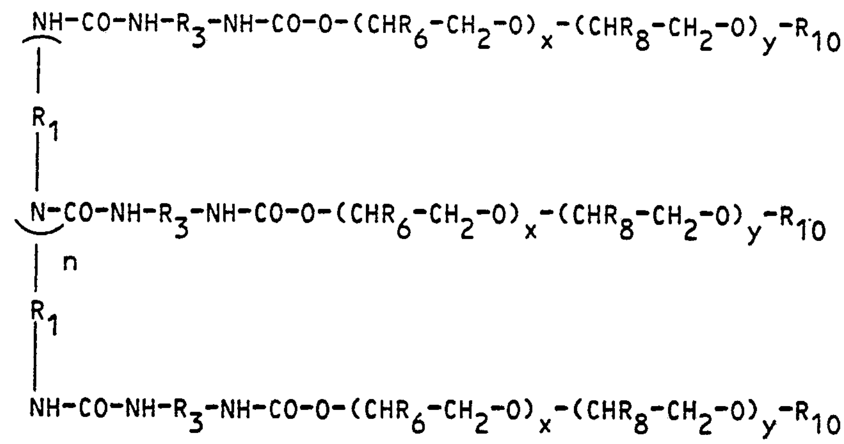

dans laquelle:

$R_1$ représente un groupe alkylène en $C_2$ à $C_7$ ou cycloalkylène en $C_5$ à $C_7$,

$R_3$ représente un groupe alkylène en $C_6$ à $C_{20}$ ou un groupe cycloalkylène ou arylène en $C_6$ à $C_{20}$, éventuellement substitué par un groupe alkyle en $C_1$ à $C_4$ ou par de l'halogène,

$R_6$ et $R_8$ représentent chacun un atome d'hydrogène ou un groupe methyle,

$R_{10}$ représente un groupe alkyle en $C_1$ à $C_6$,

n représente les nombres 0 à 5, et

x et y représentent les nombres 5 à 100,

les motifs ($CHR_6$-$CH_2$-O) et ($CHR_8$-$CH_2$-O) étant présents en blocs ou longues séquences, étant répartis statistiquement ou étant partiellement sous forme de blocs et partiellement en répartition statistique.

3. Agent de thermosensibilisation selon la revendication 2, dans lequel:

$R_1$ represente un groupe alkylène en $C_2$ à $C_3$,

$R_3$ représente un groupe alkylène en $C_6$ à $C_{13}$, un groupe cycloalkylène en $C_6$ à $C_{13}$ ou arylène en $C_6$ à $C_{13}$, éventuellement substitués par un groupe méthyle,

$R_6$ représente un atome d'hydrogène,
$R_8$ représente un groupe méthyle,
n représente les nombres 1 à 5,
x représente les nombres 5 à 50, et
y représente les nombres 5 à 40,
et les motifs ($CH_2$-$CH_2$-O) et ($CH(CH_3)$-$CH_2$-O) sont présents en répartition statistique ou partiellement en blocs et partiellement en repartition statistique.

4. Procédé pour préparer des composés selon la revendication 1, caractérisé en ce qu'on fait réagir des polyisocyanates à valence (m + 1) avec des polyéthers de formule:

$$HO-(CHR_6-CHR_7-O)_x-(CHR_8-CHR_9-O)_y-R_{10}$$

et des polyamines de formule:

$$NH_2-(R_1-NH)_n-R_2-NH_2,$$

dans lesquelles les restes $R_1$, $R_2$, $R_6$, $R_7$, $R_8$, $R_9$ et $R_{10}$ ainsi que les nombres m, n, x et y ont le sens indiqué ci-dessus.

5. Procédé pour préparer des latex rendus thermosensibles, procédé caractérisé en ce qu'on ajoute aux latex des composés selon la revendication 1.

6. Procédé selon la revendication 5, caractérisé en ce qu'on ajoute au latex l'agent de thermosensibilisation en des quantites de 1 à 20 % en poids, par rapport au polymère.